**Europäisches Patentamt**

**European Patent Office**

**Office européen des brevets**

(19)

(11) Veröffentlichungsnummer: **0 062 085**
**B1**

(12) ## EUROPÄISCHE PATENTSCHRIFT

(45) Veröffentlichungstag der Patentschrift:
**14.08.85**

(51) Int. Cl.⁴: **C 12 P 21/00, A 61 K 45/02 //**
**C12R1/91**

(21) Anmeldenummer: **81102606.1**

(22) Anmeldetag: **07.04.81**

(54) **Verbessertes Verfahren zur Herstellung von Humaninterferon aus lymphoblastoiden Zellen.**

(43) Veröffentlichungstag der Anmeldung:
**13.10.82 Patentblatt 82/41**

(45) Bekanntmachung des Hinweises auf die Patenterteilung:
**14.08.85 Patentblatt 85/33**

(84) Benannte Vertragsstaaten:
**BE CH FR GB IT LI LU NL SE**

(73) Patentinhaber: **Dr. Karl Thomae GmbH, Postfach 1755,**
**D-7950 Biberach (Riss) (DE)**

(72) Erfinder: **Swetly, Peter, Dr., Hietzinger**
**Hauptstrasse 40B/9, A-1130 Wien (AT)**
Erfinder: **Bodo, Gerhard, Dr., Belghofergasse 27,**
**A-1120 Wien (AT)**
Erfinder: **Adolf, Günther, Johannagasse 20/7,**
**A-1050 Wien (AT)**

(56) Entgegenhaltungen:
**EP - A - 0 000 520**
**EP - A - 0 005 476**

**CHEMICAL ABSTRACTS, Band 92, Nr. 19, 12. Mai 1980,
Seite 440, Nr. 161977p Columbus, Ohio, U.S.A. H.
HEREMANS et al.: "Interferon production and virus
replication in lymphoblastoid cells infected with different
viruses. Brief report"
CHEMICAL ABSTRACTS, Band 94, Nr. 17, 27. April 1981,
Seite 550, Nr. 137271z Columbus, Ohio, U.S.A. N. FUJII:
"Studies on establishment and maintenance of
persistent infection with measles virus in NC-37 cell. II.
Effect of DNA-synthesis-suppressing factor (DSF) on the
production of interferon in NC-37 cell infected with
measles virus"
N.B. FINTER: "Interferons and interferon inducers",
1973, North-Holland publishing company, Chapter 5,
Seiten 73-105 Amsterdam, NL. MONTO HO: "Factors
influencing interferon production"**

## Beschreibung

Die Herstellung von Humaninterferon aus Zellen menschlichen Ursprungs hat aufgrund der medizinischen Bedeutung der Interferone bei der Behandlung akuter und chronischer Virusinfektionen, sowie bei der Krebstherapie weltweite Beachtung gefunden. Die hierfür benötigten Mengen von Interferon können jedoch durch die bisher bekannten Herstellungsverfahren nicht zur Verfügung gestellt werden, da nur niedrige Konzentrationen von Humaninterferon erreicht werden.

Die Herstellung der Interferone erfolgt bei den bisher bekannten Verfahren durch Behandlung der Zellen mit einem Interferoninduktor (z.B. Virus oder Nukleinsäure) und anschliessende Inkubation der behandelten Zellen in Zellkulturmedium. Die Interferonausbeute kann hierbei gesteigert werden, wenn Zellen vor der Induktion mit kleinen Mengen Interferon, dem sogenannten Priming (siehe Stewart et al. in J. Virol., $\underline{7}$, 792 (1971)), behandelt werden.

Da Interferone speziesspezifisch wirksam sind, können zur Herstellung von am Menschen wirksamen Interferonen nur Humanzellen verwendet werden. Dafür kommen sowohl Zellen des Fibroblastentyps in Frage, welche auf der Oberfläche kultiviert werden, als auch Zellen hämatopoetischen Ursprungs, welche in Suspension kultiviert werden können. Zur letzt genannten Gruppe zählen humane lymphoblastoide Zellen, welche den Vorteil besitzen, unbeschränkte Zeit in Suspensionskultur mit einem Minimum an technischer Manipulation propagiert werden zu können. Besonders geeignet erwies sich die Zellinie Namalwa (Strander H., Mogensen, K.E. and Cantell, K.: Production of human lymphoblastoid Interferon in Journal of Clinical Microbiology $\underline{1}$, 116 (1975)). Diese Zellen produzieren nach Induktion mit Sendai Virus oder Newcastle Disease Virus $10^2$–$10^4$ Internationale Einheiten (IE) Interferon/ml.

Desweiteren ist bekannt (siehe EP-A-0.000.520), dass durch Vorbehandlung von Namalwa Zellen mit kurzkettigen Fettsäuren vor Priming und Virusinduktion sich die Produktion von Interferon steigern lässt und Werte bis zu 6 × $10^4$ IE/ml erreicht.

Überraschenderweise wurde nun gefunden, dass durch Zugabe einer oder mehreren Stimulatorsubstanzen aus der Reihe der kurzkettigen Fettsäuren zu NC-37-Zellen, die Interferonproduktion wesentlich stärker gesteigert werden kann als bei Namalwa Zellen und dass Interferonmengen von $10^5$ IE/ml produziert werden können, wobei die Reinheit einer derartigen Präparation in bezug auf Gesamtprotein der Erntelösung bei $10^6$ IE/mg Protein ohne Verwendung weiterer Reinigungsschritte liegt.

Als Stimulatorsubstanz hat sich insbesondere eine gesättigte, aliphatische Carbonsäure, z.B. eine Alkansäure mit 3–6 Kohlenstoffatomen, wie die Propionsäure, n-Buttersäure, Isobuttersäure, Valeriansäure, Capronsäure und deren Salze mit anorganischen oder organischen Basen, insbesondere jedoch deren Alkalisalze, bewährt.

Die NC-37-Zellen wurden von Durr, F.E., Monroe, J.H. Schmitter, K., Traul, K.A., und Hirsaut (Y. International Journal of Cancer $\underline{6}$, 436 (1970)) aus peripheren Blutzellen eines Epstein-Barr Virus (EBV) seroaktiven, gesunden Donors, isoliert und sind bei Associated Biomedical System Inc., Buffalo (USA), erhältlich.

NC-37 Zellen werden in einem Wachstumsmedium, vorzugsweise dem Medium RPMI 1640 (Fa. Gibco, USA oder Fa. Flow, Grossbritannien) kultiviert, welches als Zusätze 1–10 Vol%, vorzugsweise jedoch 2–6 Vol% vorgereinigtes Humanserum enthält. Hierbei können alternativ oder zusätzlich zu Humanserum auch Serum oder Serumfraktionen anderer Spezies verwendet werden, z.B. fötales Rinderserum in einer Konzentration von 2–15 Vol%. Als weitere Zusätze können Lactalbuminhydrolysat – und Antibiotica – dem Wachstumsmedium zugesetzt werden.

Die derart kultivierten Zellen werden in Suspension bei einer Dichte von 0.2 – 5 × $10^6$ Zellen/ml, vorzugsweise aber bei einer Dichte von 0.4 – 1 × $10^6$ Zellen/ml, mit einer Stimulatorverbindung, z.B. einem Alkalisalz der n-Buttersäure, in einer Endkonzentration von zweckmässigerweise 1 mMol/l versetzt und zweckmässigerweise bei einer Temperatur von 33–39 °C inkubiert. Nach einem Zeitraum von 6–72 Stunden, vorzugsweise jedoch von 24–48 Stunden, werden die Zellen durch Zentrifugation gewonnen und zweckmässigerweise bei einer Dichte von 1 – 5 × $10^7$ Zellen/ml in Medium, mit oder ohne Stimulatorsubstanz supendiert. Diese Suspension kann nun wahlweise einem «Priming» mit Interferon unterworfen werden: Dazu wird die Zellsuspension mit 1–1000 IE/ml Humaninterferon, vorzugsweise jedoch 100–500 IE/ml Humaninterferon, versetzt und bei 37 °C inkubiert. Die Zugabe des Interferons 5 Minuten vor Zugabe des Interferoninduktors hat sich dabei als ausreichend erwiesen. Die Zellsuspension, mit oder ohne «Priming» wird beispielsweise mit Sendaivirus bei einer Konzentration von $2^9$ – $2^{13}$ Hämagglutinierende Einheiten HAU/ml, bevorzugt jedoch mit $2^{10}$–$2^{11}$ HAU/ml, als Interferoninduktor infiziert und bei 33–39 °C inkubiert. Nach ungefhärt 1–3 Stunden werden die infizierten Zellen durch Zentrifugation gesammelt und bei einer Dichte von 1 –5 × $10^6$ Zellen/ml in einem serumfreien Zellkulturmedium, z.B. in «Minimum Essential Medium Eagle» (Fa. Gibco, USA oder Fa. Flow, Grossbritannien), für einen Zeitraum von 10–24 Stunden, vorzugsweise aber 18–20 Stunden, bei 33–39 °C und einem pH Wert des Gewebekulturmediums von 6,7–8,0, vorzugsweise jedoch von 7,3, inkubiert. Die erhaltene Lösung des lymphoblastoiden Rohinterferons wird durch Zentrifugation von den NC-37-Zellen abgetrennt und zur Inaktivierung restlicher Induktorviren durch Zugabe von Mineralsäure, beispielsweise von Salzsäure auf einen pH-Wert von vorzugsweise 2,0 eingestellt und mehrere Tage z.B. 1–5 Tage bei 0–4 °C inkubiert.

Zur weiteren Reinigung kann die so gewonnene Lösung von lymphoblastoidem Rohinterferon nach Belieben literaturbekannten Reinigungsschritten unterzogen werden.

Die nachfolgenden Beispiele sollen die Erfindung näher erläutern, jedoch nicht dem Umfang der Erfindung einschränken.

Vorbemerkung:

NC-37-Zellen werden in Suspensionskultur in Schüttelkulturen, Rollkulturen oder in einem Fermentor vermehrt. Hierbei wird als Wachstumsmedium das Medium RPMI 1640 verwendet, dem 0,1 Vol% Lactalbuminhydrolysat, 2–4 Vol% vorgereinigtes Humanserum und zusätzlich oder alternativ 2–5% fötales Rinderserum zugegeben ist. Weiters enthält das Wachstumsmedium Antibiotica; entweder Penicillin-Streptomycin oder Gentamycin. Das vorgereinigte Humanserum wird durch Fällung eines Teiles der Serumproteine mit 6% Polyäthylenglykol 6000 hergestellt (Inglot et al. Acta Virol. 19, 250 (1975)). Alternativ wird als Wachstumsmedium das «Minimum «Essential Medium Eagle» mit 5–10% fötalem Rinderserum und Antibiotikazusatz verwendet. Das Sendai Virus wird in embryonierten Hühnereiern vermehrt, die Reinigung erfolgt durch Hochgeschwindigkeitszentrifugation und anschliessende Suspension in Wachstumsmedium mittels Ultraschall-Behandlung. Das Sendai Virus wird bei −70 °C aufbewahrt.

Beispiel 1

NC-37-Zellen in exponentiellem Wachstumsstadium wurden in Rollflaschenkulturen bei einer Dichte von $4 \times 10^5$ Zellen/ml in 2000 ml RPMI 1640 Wachstumsmedium 24 Stunden bei 37 °C kultiviert, und anschliessend in Gegenwart von 1 mMol/l Natrium-n-butyrat im Wachstumsmedium weitere 40 Stunden bei 37 °C inkubiert. Die Zellen wurden durch Zentrifugation ($700 \times g$, 15 Minuten) gesammelt und bei einer Dichte von 20 $\times 10^6$ Zellen/ml in 66 ml Wachstumsmedium bei 37 °C mit 2 ml Sendai Virus (HAU $= 2^{14}$/ml) zwei Stunden bei 37 °C geschüttelt. Die auf diese Weise induzierten Zellen wurden durch Zentrifugation gesammelt und bei einer Dichte von 2.0 $\times 10^6$ Zellen/ml in 660 ml serumfreiem Eagle's-Minimum Essential Medium (mit Earle Salzen) bei 37 °C 20 Stunden lang bei pH 7,3 in Rollerflaschen inkubiert. Die Rollgeschwindigkeit betrug 0,5 Umdrehungen/Minute. Die Rohlösung (= Zellüberstand) wurde nach Abzentrifugieren der Zellen ($200 \times g$, 15 Minuten) bei einer Temperatur von maximal 10 °C mit 5 n Salzsäure auf pH 2 eingestellt und 4 Tage bei 4 °C zur Inaktivierung des Sendai Virus gelagert. Ein gleicher Versuchsansatz, jedoch ohne Zusatz von Natrium-n-butyrat, diente zur Bestimmung der Interferonproduktionssteigerung durch Stimulatorsubstanzen in NC-37 Zellen.

Die nachfolgende Tabelle enthält die gefundene Ausbeutesteigerung an Interferon in NC-37-Zellen nach Zugabe von 1 mMol/l Natrium-n-butyrat.

| | KonzentrationNatrium-n-butyrat (mMol/l) | |
| --- | --- | --- |
| | 0 | 1 |
| Volumen der NC-37-Zellsuspension nach Induktion (ml | 660 | 660 |
| Interferonproduktion (Internationale Einheiten IE/ml) | 30 | 44 200 |
| Produktionssteigerung (Faktor) | 1 | 1 470 |
| Gesamtproteingehalt in der Rohinterferonpräparation (mg/ml) | 0.037 | 0.037 |
| spezifische Aktivität des Rohinterferons (IE Interferon/mg Protein) | 810 | 1.215.420 |

Beispiel 2

Um die Interferonproduktion in NC-37-Zellen mit derjenigen in Namalwa Zellen mit und ohne Zusatz von Stimulatorsubstanzen zu vergleichen, wurde folgendes Beispiel durchgeführt.

In Parallelkulturen wurden NC-37-Zellen und Namalwa Zellen bei einer Zelldichte von 0,25 $\times 10^6$ Zellen/ml (Gesamtvolumen je 500 ml) in Minimum Essential Medium Eagle, welches mit 10 Vol% fötalem Rinderserum kompletiert war, entweder mit 1 mMol/1 Natrium-n-butyrat versetzt, oder unbehandelt 26 Stunden bei 37 °C inkubiert. Die Zellen wurden durch Zentrifugation gesammelt ($700 \times g$, 15 Minuten) und bei einer Dichte von 5 $\times 10^7$ Zellen/ml bei 37 °C 2 Stunden in Rollkulturflaschen mit Wachstumsmedium, welches $2^{10}$ HAU Sendai/Virus/ml enthielt, inkubiert. Die derart induzierten Zellen wurden durch Zentrifugation gesammelt und bei einer Dichte von 5 $\times 10^6$ Zellen/ml in serumfreiem Minimum Essential Medium Eagle bei 37 °C 20 Stunden lang bei pH $= 7.3$ inkubiert. Die Rohlösung wurden nach Abzentrifugieren der Zellen, analog zu Beispiel 1 behandelt.

Das Ergebnis von Beispiel 2 ist in der nachfolgenden Tabelle zusammengefasst und zeigt im direkten Vergleich die Überlegenheit von NC-37 Zellen gegenüber Namalwa Zellen für die Produktion von lymphoblastoidem Humaninterferon: Sowohl die Steigerung der Interferonproduktion durch Natrium-n-butyratbehandlung als auch die absolute Ausbeute an Interferon liegt in NC-37 Zellen höher als in Namalwa-Zellen.

| Konzentration Natrium-n-butyrat m Mol/l | Interferonproduktion (IE/10^6 Zellen): | | Steigerungsfaktor der Interferonproduktion | |
|---|---|---|---|---|
| | Namalwa | NC-37 | Namalwa | NC-37 |
| 0 | 280 | 16 | 1 | 1 |
| 1 | 7 270 | 21 000 | 26 | 1 300 |

Beispiel 3

Zur Bestimmung des «Priming»-Effekts auf die Interferonproduktion in NC-37-Zellen wurde folgendes Beispiel durchgeführt:

NC-37 Zellen wurden wie in Beispiel 1 kultiviert und bei einer Zelldichte von 0.55 × 10^6 Zellen/ml, (1000 ml) mit 1 mMol/l Natrium-n-butyrat 48 Stunden bei 37 °C inkubiert. Die Zellen wurden durch Zentrifugation geerntet (700 × g, 15 Minuten), in 22 ml Wachstumsmedium resuspendiert und in zwei Aliquote zu 11 ml geteilt. Zu einem der Aliquote wurden 500 IE/ml Humaninterferon zugesetzt und 5 Minuten bei 37 °C geschüttelt («Priming»), das zweite Aliquot wurde unbehandelt gelassen.

Zu jedem der Aliquote wurden 0,35 ml Sendai Virus Suspension (2^14 HAU/ml) zugesetzt und 2 Stunden bei 37 °C unter Schütteln inkubiert. Die derart induzierten Zellen wurden durch Zentrifugation gesammelt und bei einer Dichte von 2.0 × 10^6 Zellen/ml in serumfreiem Minimum Essential Medium Eagle bei 37 °C 18 Stunden bei pH = 7.3 inkubiert. Die Rohlösungen (nach Abzentrifugieren der Zellen bei 2000 × g, 15 Minuten) wurden wie in Beispiel 1 behandelt.

Die Ergebnisse sind in der nachfolgenden Tabelle zusammengefasst und zeigen, dass durch «Priming» mit Interferon in NC-37 Zellen eine Steigerung der Interferonproduktion auf 10^5 IE/ml Rohlösung erreicht werden konnte:

Effekt des «Primings» mit Humaninterferon auf die Interferonausbeute in NC-37 Zellen:

| «Priming» mit IE Interferon/ml | Interferonproduktion in NC-37 Zellen | |
|---|---|---|
| | IE/ml Rohlösung | IE/mg Protein in der Rohlösung |
| 0 | 67 900 | 622 500 |
| 500 | 97v000 | 970 000 |

**Patentansprüche**

1. Verfahren zur Herstellung von Humaninterferon, dadurch gekennzeichnet, dass in Suspension kultivierte NC-37-Zellen mit einer oder mehreren Stimulatorsubstanzen aus der Reihe der kurzkettigen Fettsäuren oder deren Salzen behandelt werden, die Interferonproduktion durch virale Induktoren angeregt wird und das so gebildete Interferon abgetrennt und/oder gereinigt wird.

2. Verfahren nach Anspruch 1, dadurch gekennzeichnet, dass die verwendeten lymphoblastoiden Zellen vor der Induktion mit einer «Priming»-Substanz behandelt werden.

3. Verfahren nach den Ansprüchen 1 und 2, dadurch gekennzeichnet, dass als Stimulatorsubstanzen Alkansäuren mit 3 bis 6 Kohlenstoffatomen und deren Salze verwendet werden.

4. Verfahren nach den Ansprüchen 1–3, dadurch gekennzeichnet, dass als Stimulatorsubstanz n-Buttersäure und als «Priming»-Substanz Humaninterferon verwendet wird.

5. Verfahren gemäss Anspruch 1–4, dadurch gekennzeichnet, dass die Endkonzentration der Stimulatorsubstanz 0.5–5 mMol/l beträgt.

6. Verfahren gemäss den Ansprüchen 1–5, dadurch gekennzeichnet, dass Humaninterferon als «Priming»-Substanz vor und/oder während der Virusinduktion zugesetzt wird.

7. Verfahren gemäss den Ansprüchen 1–6, dadurch gekennzeichnet, dass als Interferoninduktor ein viraler Induktor verwendet wird.

**Revendications**

1. Procédé de préparation d'interféron humain, caractérisé en ce que des cellules NC-37 cultivées en suspension sont traitées par une ou plusieurs substances stimulantes de la série des acides gras à chaîne courte ou leurs sels, en ce que la production d'interféron est stimulée par des inducteurs viraux et en ce que l'interféron ainsi formé est séparé et/ou purifié.

2. Procédé suivant la revendication 1, caractérisé en ce que les cellules lymphoblastoïdes utilisées sont traitées avant l'induction avec une substance de «priming».

3. Procédé suivant les revendications 1 et 2, caractérisé en ce qu'on utilise comme substances stimulantes des acides alcanoïques avec 3 à 6 atomes de carbone ou leurs sels.

4. Procédé suivant les revendications 1–3, caractérisé en ce qu'on utilise comme substance stimulante de l'acide n-butyrique et comme substance de «priming» de l'interféron humain.

5. Procédé suivant la revendication 1–4, caractérisé en ce que la concentration finale de la substance stimulante est de 0,5–5 mmoles/l.

6. Procédé suivant les revendications 1–5, caractérisé en ce que de l'interféron humain est ajouté comme substance de «priming» avant et/ou pendant l'induction par virus.

7. Procédé suivant les revendications 1–6, caractérisé en ce qu'on utilise comme inducteur d'interféron un inducteur viral.

**Claims**

1. Process for preparing human interferon, characterised in that NC-37 cells cultivated in suspension are treated with one or more stimulator substances selected from the series of short-chain fatty acids or salts thereof, the production of interferon is stimulated by viral inductors and the interferon thus formed is separated off and/or purified.

2. Process as claimed in claim 1, characterised in that the lymphoblastoid cells used are treated with a «priming» substance before the induction.

3. Process as claimed in claims 1 and 2, characterised in that alkanoic acids with 3 to 6 carbon atoms and the salts thereof are used as stimulator substances.

4. Process as claimed in claims 1 to 3, characterised in that n-butyric acid is used as the stimulator substance and human interferon is used as the «priming» substance.

5. Process as claimed in claims 1 to 4, characterised in that the final concentration of stimulator substance is 0.5 to mmol/l.

6. Process as claimed in claims 1 to 5, characterised in that human interferon is added ad the «priming» substance before and/or during the virus induction.

7. Process as claimed in claims 1 to 6, characterised in that a viral inductor is used as the interferon inductor.